# EUROPEAN PATENT SPECIFICATION

(11) **EP 3 380 183 B1**
(45) Date of publication and mention of the grant of the patent: **19.05.2021**
(21) Application number: 16829142.5
(22) Date of filing: 25.11.2016
(51) Int. Cl.: A61M 25/10, A61M 39/02, A61M 39/04

(54) **A DEVICE FOR EASY VASCULAR ACCESS**
VORRICHTUNG FÜR EINFACHEN GEFÄSSZUGANG
DISPOSITIF POUR ACCÈS VASCULAIRE AISÉ

(30) Priority: 25.11.2015 TR 201514922
(43) Date of publication of application: 03.10.2018
(73) Proprietor: Buharalioglu, Yavuz Selim, 34744 Istanbul (TR)
(72) Inventor: Buharalioglu, Yavuz Selim, 34744 Istanbul (TR)
(74) Representative: Cayli, Hülya
(86) International application number: PCT/TR2016/050461
(87) International publication number: WO 2017/091180

(56) References cited:
- WO-A1-96/25197
- US-A1- 2001 007 931
- US-A1- 2004 147 867
- US-A1- 2007 083 156

## Description

### Relevant Technical Field

The present invention relates to a device for easy vascular access, required especially for dialysis of patients with chronic kidney disease.

### Background Art

In various diseases, vascular access is required to deliver medicaments, serum and fluids such as fresh blood directly into the blood vessel or to collect blood therefrom. In conventional applications, vascular access is achieved using plastic pieces with needles, also called as vascular access devices. Said vascular access is in the form of a conduit, one part of which left outside of the patient's body and another part of which is inserted into at least one vessel of the patient. The vascular access in conventional applications provides access to the vessel for a short period of time (for example, at most 10-15 days). At the end of said period of time, the vascular access must be removed in order to avoid infection.

Patients suffering from chronic kidney disease must be dialyzed at certain intervals (for example, every 2-3 days). The dialysis machines are for example connected to a vessel of the patient in order to collect blood from the vessel it is connected and clean it so as to be re-introduced into the vessel of the patient. Therefore, every 2-3 days, vascular access is necessary for the patients who will be dialyzed. Since the vascular access system in conventional applications is maintained in the body of the patient for a short period of time, a new vascular access is required practically for every dialysis process. This, however, is painful for the patient and also, since it results in permanent damages on blood vessels, it is not possible to use it for a long period of time.

In the prior art, there are provided some applications that may be used for a longer period of time than conventional vascular access devices, in order to eliminate the above-mentioned drawbacks. One of these embodiments is arteriovenous fistula (AVF) disclosed in DE3604151A1. However, since a connection is established between the artery and the vein in arteriovenous fistula application, this may cause such conditions as heart failure. Furthermore, since the amount of blood leading to the periphery (limbs such as hands, feet etc.) is limited, ischemia may occur, and venous structures that are not accustomed to high pressure may expand and calcified, thereby leading to arterialization. In the prior art, dialysis catheters may be used as an alternative to arteriovenous fistula application. But, dialysis catheter may cause problems such as infections, blockage, and constriction of the vessel to which it is applied US2001/007931 discloses a prior art access device as in the preamble of claim 1.

### Brief Description of the Invention

With the present invention, there is provided a device for easy vascular access allowing for external access to a blood vessel. Said device for easy vascular access comprises at least a first port positioned subcutaneously and guiding a needle inserted from outside; at least a second port positioned subcutaneously and guiding a needle inserted from outside; at least one graft at least one part of which is connected to a vessel, and at least another part of which is connected to the first port and the second port, and which comprises at least one connection conduit at least one part of which is connected to the said vessel, and at least another part of which is connected to the second port and which allows the needle inserted from outside to reach to the vessel, and at least one expansion conduit at least one part of which is connected to the first port and which is expanded when filled with a fluid and thus blocks the connection conduit; and at least one needle which is inserted through the skin of the patient into the vessel via the second port.

In the device for easy vascular access according to the present invention, since all of the components are made of a tissue-compatible material and the device for vascular access is positioned subcutaneously, the device for easy vascular access can be used without a risk of infection and for a long period of time. In addition, as the permeability of the connection conduit (whether it is blocked or not) can be controlled by means of the expansion conduit, intra-graft blockage and infection can be prevented. Furthermore, due to the fact that no pieces are available in the vessel that would impair flow when not on dialysis, such problems as blockage of the patient's vessel and malfunctioning thereof are prevented.

### Object of the Invention

An object of the present invention is to provide a device for easy vascular access that can be used for a long period of time.

Another object of the present invention is to provide a device for easy vascular access that can be used with a single blood vessel (for example artery) or two blood vessels (for example an artery and a vein).

Another object of the present invention is to provide a device for easy vascular access which is easy-to-use and practical.

### Description of the Drawings

Illustrative embodiments of the device for easy vascular access according to the present invention are illustrated in the enclosed drawings, in which:
Figure 1 is a perspective view of a device for easy vascular access.
Figure 2 is a perspective view of another embodiment of the device for easy vascular access.
Figures 3a-3b are sectional perspective views of a graft used in the device for easy vascular access.
Figure 4 is a side sectional view of a second port used in the device for easy vascular access.
Figure 5 is a side sectional view of a first port used in the device for easy vascular access.
Figures 6-7 are perspective views of a needle used in the device for easy vascular access.
Figure 8 is a side sectional view of a section of the graft that is connected to the vessel, in an embodiment of the device for easy vascular access.
Figure 9 is side sectional view of a section of the graft that is connected to the vessel, in another embodiment of the device for easy vascular access.
Figure 10 is side sectional view of a section of the graft that is connected to the vessel, in another embodiment of the device for easy vascular access.
Figure 11 is a top view of the first port and the second port in an alternative embodiment of the device for easy vascular access.
Figure 12 is a side sectional view of the second port used in an alternative embodiment of the device for easy vascular access.
Figure 13 is another side sectional view of the second port used in an alternative embodiment of the device for easy vascular access.
Figure 14 is a side sectional view of an integral port used in an alternative embodiment of the device for easy vascular access.
Figure 15 is side sectional view of a section of the graft that is connected to the vessel in different conditions, in another embodiment of the device for easy vascular access.

All the parts illustrated in the drawings are individually assigned a reference numeral and the corresponding terms of these numbers are listed as follows:

| | |
|---|---|
| Blood vessel | (D) |
| Device for easy vascular access | (S) |
| Graft | (1) |
| First port | (2) |
| Second port | (3) |
| Expansion conduit | (4) |
| Connection conduit | (5) |
| Delivery chamber | (6) |
| Base | (6a) |
| Gel | (7) |
| Body | (8) |
| Delivery line | (9) |
| Needle | (10) |
| Sheath | (11) |
| Flexible coil | (12) |
| Connection lug | (13, 15) |
| Adherence member | (14) |
| Valve | (16) |
| Flap | (17) |
| First magnetic member | (18) |
| Second magnetic member | (19) |
| Interconnection | (20) |
| First piston system | (21) |
| Second piston system | (22) |
| Movement transfer arm | (23) |
| Check valve | (24) |
| Integral port | (25) |

### Description of the Invention

Especially in patients suffering from renal failure, vascular access is required at certain intervals. Therefore, vascular access devices are inserted into the vessels of the patients which allow access thereto for a certain period of time. However, the vascular access devices used in conventional applications may result in undesired situations such as limited use, infection, blockage, heart failure, ischemia, and arterialization. Therefore, with the present invention, a device for easy vascular access is provided which is free of said drawbacks.

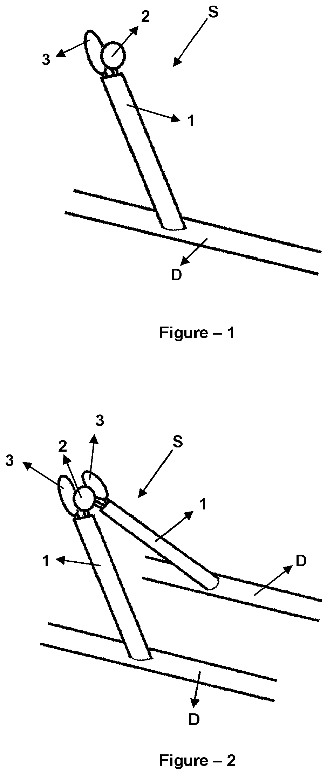

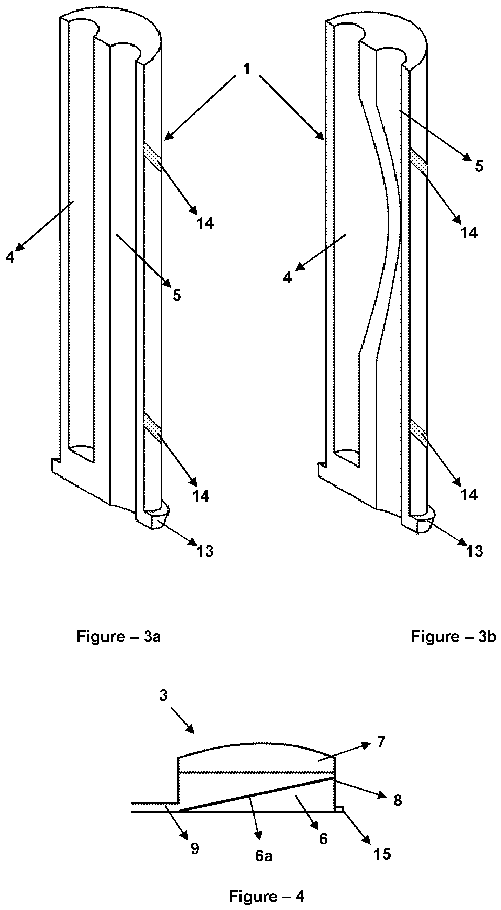

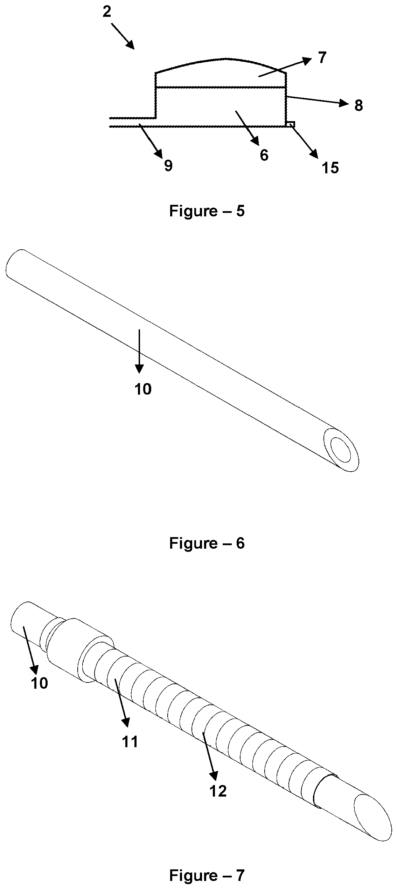

The device for easy vascular access (S) according to the present invention, as illustrated in Figures 1-2 comprises at least a first port (2) positioned subcutaneously and guiding a needle inserted from outside; at least a second port (3) positioned subcutaneously and guiding a needle and its sheath inserted from outside; at least one graft (1) at least one part of which is connected to a vessel (D) (e.g. stitched to the vessel), and at least another part of which is connected to the first port (2) and the second port (3). The graft (1) illustrated in Figures 3a and 3b comprises at least one connection conduit (5) at least one part of which is connected to the said vessel (D), at least another part of which is connected to the second port (3) and which allows the object (e.g. a needle) which is inserted from outside to reach to the vessel; and at least one expansion conduit (4) at least one part of which is connected to the first port (2) and which is expanded when filled with a fluid and thus blocks the connection conduit (5). Said device for easy vascular access (S) also comprises at least one needle (10), illustrated in detail in Figures 6 and 7, which is inserted through the skin of the patient into the vessel (D) via the second port (3) and which is preferably made of a metal material or silicon and which preferably has a tapered end. Said graft (1) is preferably made of a flexible, biocompatible material such as polytetrafluorethylene (PTFE), silicon and similar. Said graft (1) may also comprise a coating of at least one anti-microbial material (e.g. silver ion or hydroxyapatite) on its inner and/or outer surface, in order to prevent infections.

In another preferred embodiment of the invention, the device for easy vascular access (S) comprises at least one sheath (11) through which the needle (10) may be inserted. Said sheath (11) is preferably made of rigid and pliable material (e.g. silicon). Said sheath (11) is configured to be inserted through the connection conduit (5). In this embodiment of the invention, the needle (10) is located inside the sheath (11). With the needle (10) piercing the skin, the pair of sheath (11) and the needle (10) therein is advanced into the connection conduit (5) and therefrom into the vessel (D). When the needle (10) is pulled back, the sheath (11) remains under the skin and allows flow of fluid into the vessel (D). An advantage of this embodiment is that the tapered end of the needle (10) is prevented from damaging the connection conduit (5) so that the device for easy vascular access (S) is rendered durable. Said sheath (11) preferably comprises at least one flexible coil (12). Thus, durability of the sheath (11) is increased. In an embodiment of the invention with no sheath (11), said needle (10) has a hollow structure (like a syringe needle) so as to allow passage of fluid (e.g. blood or serum etc.) therethrough. In the embodiment with a sheath (11), passage of the fluid is also achieved through the sheath (11). Therefore, in the embodiment with a sheath (11), the needle (10) may have a hollow or solid structure. In an alternative embodiment of the invention, the device for easy vascular access (S) comprises at least one guide needle (not shown). Said guide needle is thicker than the needle (10) and/or its sheath (11). In an exemplary embodiment, the guide needle is used to eliminate resistance of a gel (7). Then, the sheath (11) and/or the needle (10) are introduced through the guide needle. In an alternative embodiment, the guide needle is used to apply an "embolectomy catheter" in order to clean inside of the connection conduit (5). In this embodiment, since the resistance of the gel (7) is eliminated by means of the said guide needle, it is not necessary to have the end of the needle (10) tapered. In other words, in this embodiment, the end of the needle (10) may be tapered or blunt (not tapered).

In an exemplary embodiment of the device for easy vascular access (S) according to the present invention, the device for easy vascular access (S) is connected to the patient preferably by stitching to the vessel (D) that side of the graft (1) connected thereto and positioning the first port (2) and the second port (3) subcutaneously. When there will be no access to the vessel (D) (for example, in daily/normal life of the patient), a fluid (e.g. physiological saline solution) is injected into the expansion conduit (4) by means of the first port (2) so as to expand it. As shown in figure 3b, since the connection conduit (5) is blocked when the expansion conduit (4) is expanded, external access to vessel (D) is prevented. Since the access to the vessel (D) is prevented, the blood inside the vessel (D) is also disconnected from the second port (3) and undesired situations such as infections are avoided. If it is necessary to reach to the vessel (D) (for example, if a patient is to be dialyzed), said needle (10) is inserted through the skin into the second port (3). In a preferred embodiment, the needle (10) and its sheath (11) are advanced through the connection conduit (5) (here, there is exerted a force on the needle (10) enough to eliminate blockage) so that the needle (10) and its sheath (11) are allowed to reach to the vessel directly. In this embodiment, the inner side of the connection conduit (5) may be coated with a lubricating material such as fiber or titanium in order to facilitate advancement of the needle (10) and its sheath (11). In an alternative embodiment, when the needle (10) reaches to the second port (3), a portion of the fluid inside the expansion conduit (4) is taken to eliminate blockage of the connection conduit (5), and the vessel (D) and the second port (3) (and thus the needle (10) and its sheath (11)) are connected by means of the connection conduit (5). After the relevant medical treatment is completed (for example, after the dialysis is completed), fluid is re-introduced into the expansion conduit (4) through the first port (2) so as to block the connection conduit (5). Thus, the vessel (D) is disconnected from the second port (3) in a simple and reliable manner.

In a preferred embodiment of the invention, the first port (2), as illustrated in Figure 5 in section, comprises at least one body (8) which is preferably compatible with the subcutaneous tissue such as titanium or ceramic and which forms the outer surface; at least one hollow delivery chamber (6) provided inside the said body (8) and which delivers the fluid introduced from outside into the expansion conduit (4); at least one delivery line (9) which allows to connect the delivery chamber (6) to the expansion conduit (4) and which is preferably compatible with the subcutaneous tissue such as titanium or ceramic; and at least one gel (7) provided in the delivery chamber (6), which preferably contains silicon and which isolates the graft (1) from the outer environment. Similarly, the second port (3), as illustrated in Figure 4 in section, comprises at least one body (8) which is preferably compatible with the subcutaneous tissue such as titanium or ceramic and which forms the outer surface; at least one hollow delivery chamber (6) provided inside the said body (8) and which allows to deliver the needle (10) and its sheath (11) inserted from outside into the connection conduit (5); at least one delivery line (9) which allows to connect the delivery chamber (6) to the connection conduit (5) and which is preferably compatible with the subcutaneous tissue such as titanium or ceramic; and at least one gel (7) provided in the delivery chamber (6), which preferably contains silicon and which isolates the graft (1) from the outer environment. Said second port (3) preferably comprises at least one base (6a) located in the delivery chamber (6) and inclined towards the delivery line (9). Thus, the needle (10) attached to the second port (3) is guided into the delivery line (9) in an easy and practical manner. In an alternative embodiment of the invention, the first port (2) and the second port (3) are integral. In these embodiments, the needle (10) is passed through the gel (7) into the delivery chamber. The gel (7) serves to disconnect the delivery chamber (6) from the outer environment after the needle (10) has left the first port (2) or the second port (3). Thus, after a needle is inserted into the first port (2) so as to introduce fluid into the expansion conduit (4), for example, and the needle (10) is then released from the first port (2), the fluid is prevented from leaking through the hole formed by the needle (10).

In another preferred embodiment of the invention, the device for easy vascular access (S) comprises at least two grafts (1), at least one of which is connected with the vessel (D) (e.g. an artery) and at least another of which is connected to another vessel (D) (e.g. a vein). Said device for easy vascular access (S) may either comprise at least two first ports (2) each connected with a graft (1) or a single first port (2) which is connected with at least two grafts (1) at the same time. In embodiment with a single first port (2) which is associated with two grafts (1) at the same time, said first port (2) comprises at least two delivery lines (9) each connected with the expansion conduit (4) provided in a graft (1). In an embodiment with two grafts (1), for example in a dialysis process, blood which is collected from a vessel (D) (e.g. artery) to which a graft (1) is connected is cleaned in the dialysis machine and then returned back through the vessel (D) (e.g. vein) to which another graft (1) is connected. Preferably in this embodiment, the needles (10) to be used in different grafts (1) have different colors (for example, a red needle (10) being provided in the graft (1) to be connected to the artery and a blue needle (10) being provided in the graft (1) to be connected to the vein). In this embodiment, each first port (2) and second port (3) also has different shape and/or dimensions. For example, the first port (2) is circular and the second port (3) associated with the graft (1) connected to the artery has an elongated elliptic form, and the second port (3) associated with the graft (1) connected to the vein has a short elliptic form. Thus, it is ensured that each port (2, 3) positioned under the skin is distinguished over the skin according to their shape and dimensions.

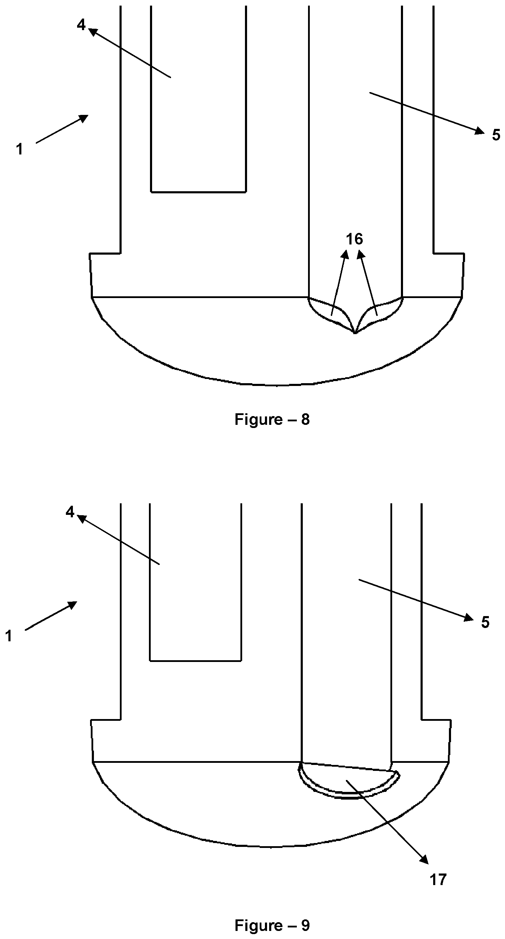

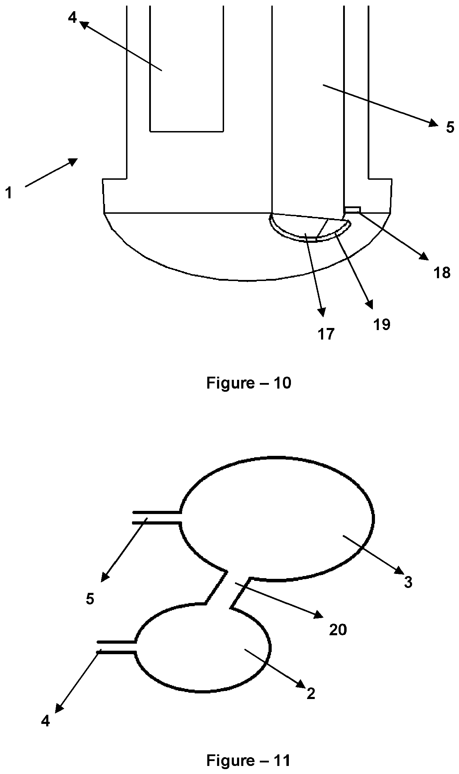

In another preferred embodiment of the invention, the device for easy vascular access (S) comprises at least one valve located on that part of the connection conduit (5) that is close to the vessel (D) and which allows one-way fluid passage (for example, which allows flow of blood just from the connection conduit (5) to the vessel (D)). In an exemplary embodiment, said valve comprises at least two valves (16) which are inclined from the connection conduit (5) towards the vessel (D) and which are flexible at the point where it is connected to the connection conduit (5), as shown in Figure 8. Said valves (16) apply force against each other and remain closed due to the flow of blood in the vessel (D), unless a pressure is applied from the connection conduit (5) in the direction of vessel. Thus, since the device for easy vascular access (S) is not used, flow of blood from the vessel (D) to the connection conduit (5) is prevented. In an alternative embodiment shown in figure 9, said valve comprises at least one flap (17) which is connected at one end with the graft (1) and which is flexible at the point where it is connected thereto. When the device for easy vascular access (S) is not used, said flap (17) closes the connection conduit (5) due to the inner pressure of the vessel (D). Thus, since the device for easy vascular access (S) is not used, flow of blood from the vessel (D) to the connection conduit (5) is prevented. In another preferred embodiment shown in Figure 10, the device for easy vascular access (S) comprises at least a first magnetic member (18) provided in the graft (1) and at least a second magnetic member (19) provided in the said flap (17). The first magnetic member (18) and the second magnetic member (19) are preferably in the form of a magnet that attracts each other. Thus, thanks to the first magnetic member (18) and the second magnetic member (19), when the graft (1) is not used, the flap (17) is ensured to be kept closed. When the graft (1) is to be used, the flap (17) is brought to an open position by means of the force applied on the needle (10) inserted through the connection conduit (5).

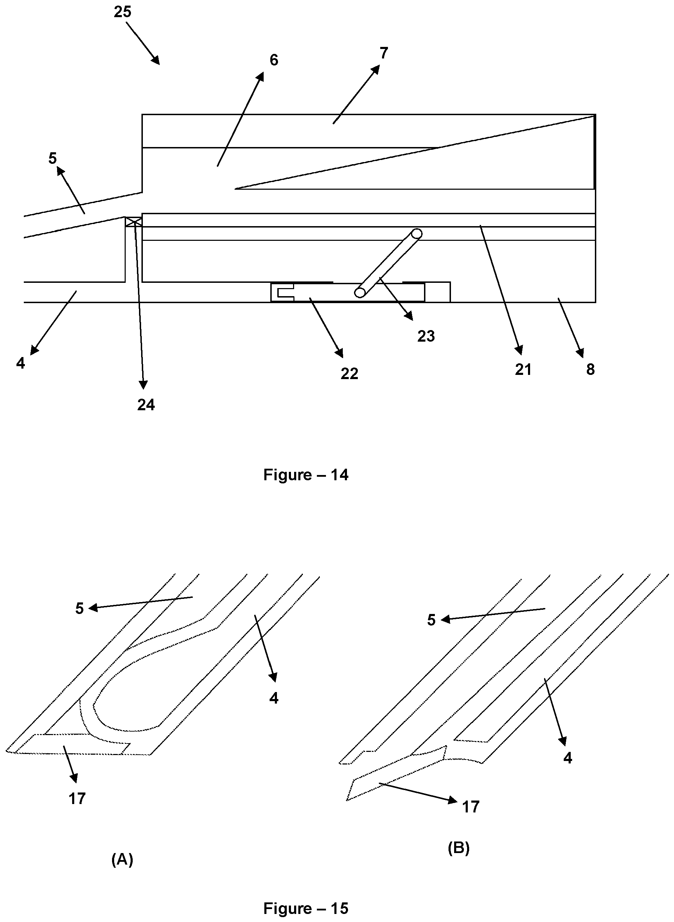

In another alternative embodiment of the invention shown in Figure 15, a section of the graft (1) that is connected to the flap (17) is flexible. In this embodiment, when the said expansion conduit (4) is expanded, the flexible section applies force on the flap (17) so as to close the connection conduit (5) (Figure 15A). When the expansion conduit (4) is restored to its initial position, the flexible section applies force on the flap (17) so as to open the connection conduit (5) (Figure 15B). Thus, if it is not necessary to reach to the vessel (D) of the patient, it is ensured that the flap (17) is remained in the position to close the connection conduit (5).

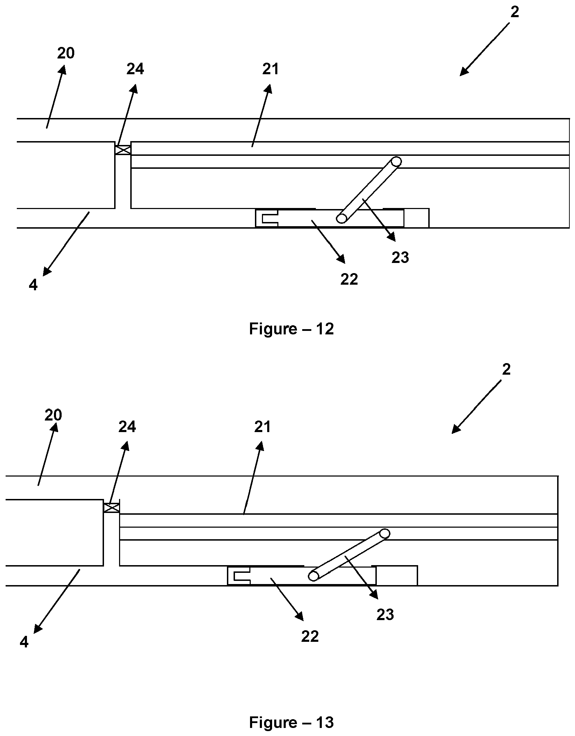

It may be uncomfortable for the patient to insert separate needles through the first port (2) and the second port (3) for different purposes. Therefore, in another preferred embodiment of the invention, the first port (2) and the second port (3) are associated, and the fluid that expands the expansion conduit (4) is conveyed from the second port (3) to the first port (2). Said device for easy vascular access (S) comprises at least one interconnection (20) which establishes a connection between the first port (2) and the second port (3), as shown in Figures 11-13. In this embodiment, the second port (2) comprises at least a first piston system (21) which is actuated by the pressure of the fluid received through the interconnection (20); at least a second piston system (22) which has a surface area lower than that of the first piston system (22) and which expands the expansion conduit (4) by applying pressure thereon; and at least one movement transfer arm (23) which transfer movement of the first piston system (21) to the second piston system (22). In an exemplary embodiment, if it is not necessary to reach to the vessel (D) of the patient, a fluid (e.g. physiological saline solution) is introduced into the second port (3) by means of the needle (10). The fluid is passed through the interconnection (20) into the first port (2) and applies pressure on the first piston system (21) to move it as shown in Figure 13. Said movement is transferred to the second piston system (22) by means of the movement transfer arm (23). The second piston system (22) is thus actuated and expands the expansion conduit (4). Since the surface area of the second piston system (22) is lower than that of the first piston system (22), the second piston system (22) is actuated in a simple manner even by a low pressure of the fluid coming from the second port (3), so that the expansion conduit (4) is expanded. When it is necessary to establish an access to the vessel (D) of the patient, the fluid inside the first port (2) is initially collected by means of a needle inserted into the second port (3). Since the pressure inside the first port (2) is reduced, the first piston system (21) and the second piston system (22) are returned to their initial position, as shown in Figure 12. Thus, the expansion conduit (4) also returns to its position wherein passage through the connection conduit (5) is allowed. Here, said first piston system (21) preferably comprises at least one elastic membrane and at least one rigid plate (for example, made of material like titanium) which is connected with the elastic membrane. In this embodiment, the device for easy vascular access (S) also comprises at least one check valve (24) permitting one-way passage of the fluid from the second port (3) into the expansion conduit (4) (that is, preventing passage of fluid from the expansion conduit (4) to the second port (3)). With the said check valve (24), the pressure of the expansion conduit (4) is maintained at the desired level.

In another preferred embodiment of the invention, the device for easy vascular access (S) comprises at least one integral port (25) accommodating the first port (2) and the second port (3), in order to provide the function of the first port (2) and the second port (3). The integral port (25), which is illustrated in detail in Figure 14, is connected to the expansion conduit (4) at at least one end thereof, and at another end, is connected to the connection conduit (5). In this embodiment, the integral port (25) comprises at least one body (8) which is preferably compatible with subcutaneous tissue such as titanium or ceramic and which forms the outer surface; at least one hollow delivery chamber (6) provided inside the said body (8); at least a first piston system (21) actuated by the pressure of the fluid introduced into the delivery chamber (6); at least a second piston system (22) having a surface area lower than that of the first piston system (22) and applies pressure on the expansion conduit (4) so as to expand it; and at least one movement transfer arm (23) which transfers the movement of the first piston system (21) to the second piston system (22). The said integral port (25) may also comprise at least one check valve (24) permitting one-way passage of the fluid from the delivery chamber (6) into the expansion conduit (4) (that is, preventing passage of fluid from the expansion conduit (4) to the delivery chamber (6)).

In another preferred embodiment of the invention, the device for easy vascular access (S) comprises at least one connection member (not shown) located at a section where the first port (2) and/or the second port (3) is connected to the graft (1) and which connects the first port (2) and/or the second port (3) to the graft (1) in a reliable manner and which is attached on the graft (1) so as to compress it. Said connection member preferably has a plastic ring form. In another preferred embodiment, the device for easy vascular access (S) comprises at least one perforated connection lug (15) located at the bottom of the first port (2) and/or the second port (3) and which ensures a reliable connection (i.e. stitching) of the first port (2) and/or the second port (3) to the subcutaneous tissue.

In another preferred embodiment of the invention, said graft (1) is inclined at that part connected to the vessel (D) (for example, it has an angle of 30°- 35° between the vessel (D) and the graft (1)). Thus, it is ensured that the graft (1) extends from the vessel (D) to the skin at an appropriate angle. In another preferred embodiment of the invention, the device for easy vascular access (S) comprises at least one connection lug (13) located at that part of the graft (1) which is connected to the vessel (D) and which ensures a reliable connection (i.e. stitching) of the graft (1) to the vessel (D). In another preferred embodiment of the invention, the device for easy vascular access (S) comprises at least one fibrous adherence member (14) which preferably surrounds the graft (1) and allows adherence of the graft (1) to the tissues. With the said adherence member (14), the graft (1) is positioned inside the tissue in a reliable manner.

In the device for easy vascular access (S) according to the present invention, since all of the components are made of a tissue-compatible material and it is positioned subcutaneously, the device for easy vascular access (S) can be used without a risk of infection and for a long period of time. In addition, since the permeability of the connection conduit (5) (whether it is blocked or not) can be controlled by means of the expansion conduit (4) and/or the valve (16) or the flap (17), intra-graft (1) blockage and infection can be prevented.

## Claims

1. A device for easy vascular access (S) allowing for external access to a vessel (D), comprising:
- at least a first port (2) positioned subcutaneously and for guiding a needle (10) inserted from outside;
- at least a second port (3) positioned subcutaneously and for guiding a needle (10) inserted from outside;
- at least one graft (1) at least one part of which is suitable to be connected to a vessel (D), and at least another part of which is connected to the first port (2) and the second port (3), and which comprises at least one connection conduit (5) at least one part of which is suitable to be connected to a vessel (D), and at least another part of which is connected to the second port (3) and which allows the needle (10) inserted from outside to reach to the vessel, and at least one expansion conduit (4) at least one part of which is connected to the first port (2) and which is expanded when filled with a fluid and thus blocks the connection conduit (5);
- at least one needle (10) which is suitable to be inserted through the skin of the patient into a vessel (D) via the second port (3) ; and
- at least one valve located on that part of the connection conduit (5) that is close to the vessel (D) and which allows one-way fluid passage
**characterised in that**, said first port (2) comprises at least one body (8) which forms the outer surface; at least one hollow delivery chamber (6) provided inside the said body (8) and which delivers the fluid introduced from outside into the expansion conduit (4); at least one delivery line (9) which connects the delivery chamber (6) to the expansion conduit (4); and at least one gel (7) for passing the needle into the delivery chamber (6).

2. A device for easy vascular access (S) according to claim 1, **characterized in that** said second port (3) comprises at least one body (8) which forms the outer surface; at least one hollow delivery chamber (6) provided inside the said body (8) and which delivers a needle (10) inserted from outside into the connection conduit (5); at least one delivery line (9) which connects the delivery chamber (6) to the connection conduit (5); and at least one gel (7) provided to access the delivery chamber (6).

3. A device for easy vascular access (S) according to claim 2, **characterized in that** the second port (3) comprises at least one base (6a) located in the delivery chamber (6) and inclined towards the delivery line (9) and thus guides the needle (10) into the delivery line (9) in an easy manner.

4. A device for easy vascular access (S) according to claim 1, **characterized by** comprising at least one sheath (11) through which the needle (10) can be inserted.

5. A device for easy vascular access (S) according to claim 1, **characterized by** comprising at least one guide needle.

6. A device for easy vascular access (S) according to claim 1, **characterized by** comprising at least two grafts (1), at least one of which is suitable to be connected with a vessel (D) and at least another one of which is suitable to be connected to another vessel (D).

7. A device for easy vascular access (S) according to claim 1, **characterized in that** said valve comprises at least two valves (16) which permit flow from the connection conduit (5) to the vessel (D), which are inclined and which are flexible at the point where it is connected to the connection conduit (5).

8. A device for easy vascular access (S) according to claim 1, **characterized in that** said valve comprises at least one flap (17) which is connected at one end with the graft (1) and which is flexible at the point where it is connected thereto.

9. A device for easy vascular access (S) according to claim 1, **characterized by** comprising at least one connection lug (13) located at that part of the graft (1) which is suitable to be connected to the vessel (D) and which ensures a reliable connection of the graft (1) to the vessel (D).

10. A device for easy vascular access (S) according to claim 1, **characterized by** comprising at least one interconnection (20) which establishes a connection between the first port (2) and the second port (3).

11. A device for easy vascular access (S) according to claim 10, **characterized in that** the second port (2) comprises at least a first piston system (21) which is actuated by the pressure of the fluid received through the interconnection (20); at least a second piston system (22) which has a surface area lower than that of the first piston system (22) and which expands the expansion conduit (4) by applying pressure thereon; and at least one movement transfer arm (23) which transfers movement of the first piston system (21) to the second piston system (22).

12. A device for easy vascular access (S) according to claim 11, **characterized in that** said first piston system (21) comprises at least one elastic membrane and at least one rigid plate which is connected with the elastic membrane.

13. A device for easy vascular access (S) according to claim 11, **characterized by** comprising at least one check valve (24) permitting one-way passage of the fluid from the second port (3) into the expansion conduit (4).

14. A device for easy vascular access (S) according to claim 1, **characterized by** comprising at least integral port (25).

15. A device for easy vascular access (S) according to claim 14, **characterized in that** said integral port (25) comprise at least one body (8) which forms the outer surface; at least one hollow delivery chamber (6) provided inside the said body (8); at least a first piston system (21) actuated by the pressure of the fluid introduced into the delivery chamber (6); at least a second piston system (22) having a surface area lower than that of the first piston system (22) and applies pressure on the expansion conduit (4) so as to expand it; and at least one movement transfer arm (23) which transfers the movement of the first piston system (21) to the second piston system (22).

## Patentansprüche

1. Vorrichtung zum einfachen Gefäßzugang (S), die einen externen Zugang zu einem Gefäß (D) ermöglicht, mit:
- wenigstens einem ersten Anschluss (2), der subkutan angeordnet ist und zur Führung einer von außen eingeführten Nadel (10) dient,
- wenigstens einem zweiten Anschluss (3), der subkutan angeordnet ist und zum Führen einer von außen eingeführten Nadel (10) dient,
- wenigstens einem Transplantat (1), von dem wenigstens eine Teil dazu geeignet ist, mit einem Gefäß (D) verbunden zu werden, und von dem ein anderer Teil mit dem ersten Anschluss (2) und dem zweiten Anschluss (3) verbunden ist und das wenigstens eine Verbindungsleitung (5), von der wenigstens ein Teil dazu geeignet ist, mit einem Gefäß (D) verbunden zu werden, und von der wenigstens ein anderer Teil mit dem zweiten Anschluss (3) verbunden ist und die es ermöglicht, dass die von außen eingeführte Nadel (10) das Gefäß erreicht, und das wenigstens eine Expansionsleitung (4) aufweist, von der wenigstens ein Teil mit dem ersten Anschluss (2) verbunden ist und die sich ausdehnt, wenn sie mit einem Fluid gefüllt wird, und die somit die Verbindungsleitung (5) blockiert,
- wenigstens einer Nadel (10), die dazu geeignet ist, durch die Haut des Patienten über den zweiten Anschluss (3) in ein Gefäß (D) eingeführt zu werden, und
- wenigstens einem Ventil, das sich an dem Teil der Verbindungsleitung (5) befindet, der nahe an dem Gefäß (D) liegt, und das den Durchgang von Fluid in einer Richtung erlaubt,
**dadurch gekennzeichnet, dass**
der erste Anschluss (2) wenigstens ein Gehäuse (8), das die äußere Oberfläche bildet, wenigstens eine hohle Zufuhrkammer (6), die in dem Gehäuse (8) angeordnet ist und die von außen zugeführtes Fluid in die Expansionsleitung (4) liefert, wenigstens eine Zufuhrleitung (9), die die Zufuhrkammer (6) mit der Expansionsleitung (4) verbindet, und wenigstens ein Gel (7) zum Hindurchführen der Nadel in die Zufuhrkammer (6) aufweist.

2. Vorrichtung zum einfachen Gefäßzugang (S) nach Anspruch 1, **dadurch gekennzeichnet, dass** der zweite Anschluss (3) wenigstens ein Gehäuse (8), das die äußere Oberfläche bildet, wenigstens eine hohle Zufuhrkammer (6), die innerhalb des Gehäuses (8) angeordnet ist und die eine von außen eingeführte Nadel (10) in die Verbindungsleitung (5) führt, wenigstens eine Zufuhrleitung (9), die die Zufuhrkammer (6) mit der Verbindungsleitung (5) verbindet, und wenigstens ein Gel (7) aufweist, das dazu vorgesehen ist, um Zugang zu der Zufuhrkammer (6) zu ermöglichen.

3. Vorrichtung zum einfachen Gefäßzugang (S) nach Anspruch 2, **dadurch gekennzeichnet, dass** der zweite Anschluss (3) wenigstens eine Basis (6a) aufweist, die sich in der Zufuhrkammer (6) befindet und die zu der Zufuhrleitung (9) hin geneigt ist und daher die Nadel (10) in einfacher Weise in die Zufuhrleitung (9) führt.

4. Vorrichtung zum einfachen Gefäßzugang (S) nach Anspruch 1, **dadurch gekennzeichnet, dass** sie wenigstens eine Hülse (11) aufweist, durch die die Nadel (10) eingeführt werden kann.

5. Vorrichtung zum einfachen Gefäßzugang (S) nach Anspruch 1, **dadurch gekennzeichnet, dass** sie wenigstens eine Führungsnadel aufweist.

6. Vorrichtung zum einfachen Gefäßzugang (S) nach Anspruch 1, **dadurch gekennzeichnet, dass** sie wenigstens zwei Transplantate (1) aufweist, von denen wenigstens eines geeignet ist, mit einem Gefäß (D) verbunden zu werden, und von denen wenigstens ein anderes dazu geeignet ist, mit einem anderen Gefäß (D) verbunden zu werden.

7. Vorrichtung zum einfachen Gefäßzugang (S) nach Anspruch 1, **dadurch gekennzeichnet, dass** das Ventil wenigstens zwei Ventile (16) aufweist, die Durchfluss von der Verbindungsleitung (5) in das Gefäß (D) erlauben, die geneigt verlaufen und die an dem Punkt, wo sie mit der Verbindungsleitung (5) verbunden sind, flexibel sind.

8. Vorrichtung zum einfachen Gefäßzugang (S) nach Anspruch 1, **dadurch gekennzeichnet, dass** das Ventil wenigstens eine Klappe (17) aufweist, die an einem Ende mit dem Transplantat (1) verbunden ist und die an dem Punkt, wo sie mit diesem verbunden ist, flexibel ist.

9. Vorrichtung zum einfachen Gefäßzugang (S) nach Anspruch 1, **dadurch gekennzeichnet, dass** sie wenigstens eine Verbindungslasche (13) aufweist, die sich an dem Teil des Transplantats (1) befindet, der dazu geeignet ist, um mit dem Gefäß (D) verbunden zu werden, und die eine verlässliche Verbindung des Transplantats (1) mit dem Gefäß (D) sicherstellt.

10. Vorrichtung zum einfachen Gefäßzugang (S) nach Anspruch 1, **dadurch gekennzeichnet, dass** sie wenigstens eine Verbindung (20) aufweist, die eine Verbindung zwischen dem ersten Anschluss (2) und dem zweiten Anschluss (3) herstellt.

11. Vorrichtung zum einfachen Gefäßzugang (S) nach Anspruch 10, **dadurch gekennzeichnet, dass** der zweite Anschluss (2) wenigstens ein erstes Kolbensystem (21), das durch Druck des Fluids betätigt wird, das durch die Verbindung (20) aufgenommen wird, wenigstens ein zweites Kolbensystem (22), das ein Oberflächengebiet unterhalb desjenigen des ersten Kolbensystems (22) hat und das die Expansionsleitung (4) durch Anwenden von Druck darauf ausdehnt, und wenigstens einen Bewegungsübertragungsarm (23) aufweist, der Bewegung des ersten Kolbensystems (21) auf das zweite Kolbensystem (22) überträgt.

12. Vorrichtung zum einfachen Gefäßzugang (S) nach Anspruch 11, **dadurch gekennzeichnet, dass** das erste Kolbensystem (21) wenigstens eine elastische Membran und wenigstens eine feste Platte aufweist, die mit der elastischen Membran verbunden ist.

13. Vorrichtung zum einfachen Gefäßzugang (S) nach Anspruch 11, **dadurch gekennzeichnet, dass** sie wenigstens ein Einwegventil (24) aufweist, das Durchfluss von Fluid in einer Richtung von dem zweiten Anschluss (3) in die Expansionsleitung (4) erlaubt.

14. Vorrichtung zum einfachen Gefäßzugang (S) nach Anspruch 1, **dadurch gekennzeichnet, dass** sie wenigstens einen integralen Anschluss (25) aufweist.

15. Vorrichtung zum einfachen Gefäßzugang (S) nach Anspruch 14, **dadurch gekennzeichnet, dass** der integrale Anschluss (25) wenigstens ein Gehäuse (8), das die äußere Oberfläche bildet, wenigstens eine hohle Zufuhrkammer (6), die innerhalb des Gehäuses (8) vorgesehen ist, wenigstens ein erstes Kolbensystem (21), das durch Druck des in die Zufuhrkammer (6) eingeführten Fluids betätigt wird, wenigstens ein zweites Kolbensystem (22), das ein Oberflächengebiet unterhalb desjenigen des ersten Kolbensystems (22) hat und Druck auf die Expansionsleitung (4) ausübt, um diese auszudehnen, und wenigstens einen Bewegungsübertragungsarm (23) aufweist, der Bewegung des ersten Kolbensystems (21) auf das zweite Kolbensystem (22) überträgt.

## Revendications

1. Dispositif pour accès vasculaire aisé (S) permettant un accès externe à un vaisseau (D), comprenant :
- au moins un premier orifice (2) positionné en sous-cutané et destiné à guider une aiguille (10) insérée depuis l'extérieur ;
- au moins un deuxième orifice (3) positionné en sous-cutané et destiné à guider une aiguille (10) insérée depuis l'extérieur ;
- au moins un greffon (1) dont au moins une partie est adaptée pour être reliée à un vaisseau (D), et dont au moins une autre partie est reliée au premier orifice (2) et au deuxième orifice (3), et qui comprend au moins un conduit de liaison (5) dont au moins une partie est adaptée pour être reliée à un vaisseau (D), et dont au moins une autre partie est reliée au deuxième orifice (3) et qui permet à l'aiguille (10) insérée depuis l'extérieur d'atteindre le vaisseau, et au moins un conduit de dilatation (4) dont au moins une partie est reliée au premier orifice (2) et qui se dilate lorsqu'il est rempli d'un fluide et bloque ainsi le conduit de liaison (5) ;
- au moins une aiguille (10) qui est appropriée pour être insérée à travers la peau du patient dans un vaisseau (D) via le deuxième orifice (3) ; et
- au moins une valve située sur la partie du conduit de liaison (5) qui est proche du vaisseau (D) et qui permet le passage de fluide unidirectionnel
**caractérisé en ce que**
ledit premier orifice (2) comprend au moins un corps (8) qui forme la surface extérieure ; au moins une chambre de distribution creuse (6) prévue à l'intérieur dudit corps (8) et qui distribue le fluide introduit depuis l'extérieur dans le conduit de dilatation (4) ; au moins une ligne de distribution (9) qui relie la chambre de distribution (6) au conduit de dilatation (4) ; et au moins un gel (7) pour faire passer l'aiguille dans la chambre de distribution (6).

2. Dispositif pour accès vasculaire aisé (S) selon la revendication 1, **caractérisé en ce que** ledit deuxième orifice (3) comprend au moins un corps (8) qui forme la surface extérieure ; au moins une chambre de distribution creuse (6) prévue à l'intérieur dudit corps (8) et qui distribue une aiguille (10) insérée depuis l'extérieur dans le conduit de liaison (5) ; au moins une ligne de distribution (9) qui relie la chambre de distribution (6) au conduit de liaison (5) ; et au moins un gel (7) prévu pour accéder à la chambre de distribution (6).

3. Dispositif pour accès vasculaire aisé (S) selon la revendication 2, **caractérisé en ce que** le deuxième orifice (3) comprend au moins une base (6a) située dans la chambre de distribution (6) et inclinée vers la ligne de distribution (9) et guide ainsi l'aiguille (10) dans la ligne de distribution (9) de manière aisée.

4. Dispositif pour accès vasculaire aisé (S) selon la revendication 1, **caractérisé en ce qu'**il comprend au moins une gaine (11) à travers laquelle l'aiguille (10) peut être insérée.

5. Dispositif pour accès vasculaire aisé (S) selon la revendication 1, **caractérisé en ce qu'**il comprend au moins une aiguille de guidage.

6. Dispositif pour accès vasculaire aisé (S) selon la revendication 1, **caractérisé en ce qu'**il comprend au moins deux greffons (1) dont au moins un est adapté pour être relié à un vaisseau (D) et dont au moins un autre est adapté pour être relié à un autre vaisseau (D).

7. Dispositif pour accès vasculaire aisé (S) selon la revendication 1, **caractérisé en ce que** ladite valve comprend au moins deux valves (16) qui permettent l'écoulement du conduit de liaison (5) vers le vaisseau (D), qui sont inclinées et qui sont flexibles au point où elle est reliée au conduit de liaison (5).

8. Dispositif pour accès vasculaire aisé (S) selon la revendication 1, **caractérisé en ce que** ladite valve comprend au moins un clapet (17) qui est relié à une extrémité avec le greffon (1) et qui est flexible au point où il est relié à celui-ci.

9. Dispositif pour accès vasculaire aisé (S) selon la revendication 1, **caractérisé en ce qu'**il comprend au moins une patte de liaison (13) située au niveau de la partie du greffon (1) qui est appropriée pour être reliée au vaisseau (D) et qui assure une liaison fiable du greffon (1) au vaisseau (D).

10. Dispositif pour accès vasculaire aisé (S) selon la revendication 1, **caractérisé en ce qu'**il comprend au moins une inter liaison (20) qui établit une liaison entre le premier orifice (2) et le deuxième orifice (3).

11. Dispositif pour accès vasculaire aisé (S) selon la revendication 10, **caractérisé en ce que** le deuxième orifice (2) comprend au moins un premier système de piston (21) qui est actionné par la pression du fluide reçu par l'inter liaison (20) ; au moins un deuxième système de piston (22) qui a une superficie inférieure à celle du premier système de piston (22) et qui dilate le conduit de dilatation (4) en appliquant une pression sur celui-ci ; et au moins un bras de transfert de mouvement (23) qui transfère le mouvement du premier système de piston (21) au deuxième système de piston (22).

12. Dispositif pour accès vasculaire aisé (S) selon la revendication 11, **caractérisé en ce que** ledit premier système de piston (21) comprend au moins une membrane élastique et au moins une plaque rigide qui est reliée à la membrane élastique.

13. Dispositif pour accès vasculaire aisé (S) selon la revendication 11, **caractérisé en ce qu'**il comprend au moins un clapet anti-retour (24) permettant le passage unidirectionnel du fluide du deuxième orifice (3) dans le conduit de dilatation (4).

14. Dispositif pour accès vasculaire aisé (S) selon la revendication 1, **caractérisé en ce qu'**il comprend au moins un orifice intégral (25).

15. Dispositif pour accès vasculaire aisé (S) selon la revendication 14, **caractérisé en ce que** ledit orifice intégral (25) comprend au moins un corps (8) qui forme la surface extérieure ; au moins une chambre de distribution creuse (6) prévue à l'intérieur dudit corps (8) ; au moins un premier système de piston (21) actionné par la pression du fluide introduit dans la chambre de distribution (6) ; au moins un deuxième système de piston (22) ayant une superficie inférieure à celle du premier système de piston (22) et appliquant une pression sur le conduit de dilatation (4) de manière à le dilater ; et au moins un bras de transfert de mouvement (23) qui transfère le mouvement du premier système de piston (21) au deuxième système de piston (22).
